# EUROPÄISCHE PATENTANMELDUNG

(11) **EP 2 018 805 A2**
(43) Veröffentlichungstag der Anmeldung: **28.01.2009**
(21) Anmeldenummer: 08012628.7
(22) Anmeldetag: 11.07.2008
(51) Int. Cl.: A01K 67/033

(54) **Verwendung von Blut, Blutplasma oder Blutserum, sowie Vorrichtung und Verfahren zur Aufzucht von Fliegen-Larven**

(30) Priorität: 21.07.2007 DE 102007034119
(71) Anmelder: AGILTERA GmbH & Co. KG, 42542 Dormagen (DE)
(72) Erfinder: Heuer, Heike Elfriede, 42542 Dormagen (DE)
(74) Vertreter: Flaccus, Rolf-Dieter

(57) **Zusammenfassung**

Die vorliegende Erfindung betrifft die Verwendung von humanem Blut, humanem Blutplasma oder humanem Blutserum zur Aufzucht von Fliegenlarven. Die Erfindung umfaßt ferner eine Vorrichtung zur Aufzucht von Fliegenlarven sowie ein Verfahren zur Aufzucht von Fliegenlarven, bei dem die Vorrichtung und/oder menschliches Blut, Blutplasma oder Blutserum verwendet wird.

## Beschreibung

Die vorliegende Erfindung betrifft die Verwendung von humanem Blut, humanem Blutplasma oder humanem Blutserum zur Aufzucht von Fliegenlarven. Die Erfindung umfaßt ferner eine Vorrichtung zur Aufzucht von Fliegenlarven sowie ein Verfahren zur Aufzucht von Fliegenlarven, bei dem die Vorrichtung und/oder menschliches Blut, Blutplasma oder Blutserum verwendet wird.

Die Larven von Calliphoriden werden in ihrem 1. und 2. Larvalstadium in der Medizin genutzt, um abgestorbenes Gewebe selektiv von lebendem Gewebe zu trennen und so eine Wundreinigung zu erzielen. Zudem wird der Wundheilungsprozeß durch das von den Larven gereinigte Gewebe deutlich beschleunigt. Für die Wundreinigung können die Larven mit verschiedenen Vorrichtungen auf der Wunde gehalten werden, wie sie beispielsweise in den Offenlegungsschriften EP 1 020 197 A1, GB 2 422 315 A oder WO 2006/029 778 A1 beschrieben sind, so daß sich die Larven nur dahin bewegen können, wo der behandelnde Arzt ihre Anwesenheit wünscht.

Die für medizinische Zwecke benötigten Calliphoriden-Larven werden unter sterilen Bedingungen auf speziellen Nährmedien aus den Eiern der Fliegen erbrütet und ernähren sich unter kontrollierten Bedingungen vom Nährsubstrat. Das Nährsubstrat für die Larven besteht üblicherweise aus einem Blut-Agar, welcher Blut von Schweinen oder Pferden sowie weitere Komponenten enthält. Ein typischer Blut-Agar für die Aufzucht von Calliphoriden-Larven besteht aus 10,0 g/l Herzextrakt, 10,0 g Tryptose, 5,0 g/l Natriumchlorid, 15,0 g/l Agar und 50 ml bis 80 ml Blut von Schweinen, Rindern, Schafen, Hammeln oder Pferden.

Calliphoriden-Larven, die therapeutischen Zwecken dienen, gelten nach internationalem Recht als Arzneimittel. Die für die Herstellung von Arzneimitteln zu verwendenden Rohstoffe unterliegen besonderen Anforderungen. So dürfen beispielsweise zur Vermeidung einer Übertragung der Bovine Spongiforme Enzephalopathie (BSE) keine von Rindern stammenden Bestandteile verwendet werden, sofern dieses vermeidbar ist. Aus diesem Grund wird für die Herstellung von Blut-Agar für die Zucht von Calliphoriden-Larven überwiegend Schweineblut oder Pferdeblut eingesetzt. Aber auch das Blut von Schweinen oder Pferden kann durch humanpathogene Prionen, Viren, Bakterien, Pilze oder Parasiten kontaminiert sein. Zudem ist zu berücksichtigen, daß Blut-Agar durch Einrühren des Blutes und der weiteren Inhaltsstoffe (siehe oben) in den nahezu erkalteten Agar hergestellt wird. Dafür wird eine rezepturgemäße Temperatur von 45 - 50 °C eingehalten. Bei dieser Temperatur und der kurzen Zeitspanne, innerhalb der diese Temperatur gehalten wird, ist nicht sichergestellt, daß die genannten Pathogene abgetötet werden.

Um eine Aufzucht von Calliphoriden-Larven zu ermöglichen, bei der die Larven nicht mit humanpathogenen Prionen, Viren, Bakterien, Pilzen oder Parasiten kontaminiert werden, wird in der DE 103 28 102 A1 vorgeschlagen, keine tierischen Bestandteile für die Zucht der Larven zu verwenden, sondern Pflanzenextrakte. Nachteilig bei diesem Verfahren ist jedoch, daß die Larven auf den in DE 103 28 102 A1 beschriebenen Medien zwar wachsen, ihre Entwicklung aber langsamer und unbefriedigend verläuft, verglichen mit einer Aufzucht auf Blut-Agar. Die Gründe hierfür könnten sein, daß die Calliphoriden-Larven Fleisch als natürliche Nahrungsquelle haben und pflanzliche Nahrung möglicherweise aufgrund ihres Stoffwechsels oder aufgrund einer für sie ungeeigneten Aminosäurezusammensetzung der pflanzlichen Extrakte nur unzureichend verwerten können.

Außerdem ist zu berücksichtigen, daß vermehrungsfähige Viren, Bakterien oder Pilze, oder pflanzliche Komponenten, die als Allergene zu Problemen bei der medizinischen Anwendung der Larven führen können, auch bei Verwendung pflanzlicher Nahrung durch die Calliphoriden-Larven auf Patienten übertragen werden können.

Der vorliegenden Erfindung lag daher die Aufgabe zugrunde, Nahrungssubstrate für im medizinischen Bereich zu verwendende Calliphoriden-Larven bereitzustellen, die eine einwandfreie Entwicklung der Eier und Larven ermöglicht, aber gleichzeitig das Risiko für die zu behandelnden Patienten, mit humanpathogenen Prionen, Viren, Bakterien, Pilzen oder Parasiten infiziert zu werden sowie mit allergenen Komponenten in Kontakt zu kommen, ausschließt oder zumindest weitgehend minimiert.

Bei der Lösung dieser Aufgabe wurde überraschenderweise gefunden, daß humanes Blut auch ohne komplexe Medienbestandteile, wie beispielsweise Herzextrakt oder Leberextrakt, ein Substrat für die Aufzucht von Fliegen-larven ist, welches eine einwandfreie Entwicklung der Fliegenlarven ermöglicht. Wenn die Fliegenlarven auf Blut humanen Ursprungs aufgezogen werden, wachsen sie sogar schneller und reinigen die Wunden besser, als Larven, die auf bekannten Nährsubstraten auf Blutbasis wachsen.

Das menschliche Blut für die Aufzucht der Fliegenlarven kann frisch gesammelt werden. Es kann jedoch nach seiner Gewinnung auch gelagert, vorzugsweise kühl gelagert, und/oder transportiert werden, bevor es für die Larvenzucht verwendet wird.

Blut, auch humanes Blut, setzt sich aus dem Blutplasma und zellulären Bestandteilen (Erythrozyten, Thrombozyten und Leukozyten, zu denen die Granulozyten, Lymphozyten und Monozyten gehören) zusammen. Überraschenderweise wurde festgestellt, daß nicht nur Vollblut, wie das alle zellulären Bestandteile enthaltende Blutplasma auch genannt wird, ein gutes Nährsubstrat für Fleischfliegen darstellt, sondern daß auch Bestandteile des Blutes vor seiner Verwendung für die Larvenzucht abgetrennt werden können, beispielsweise die roten Blutkörperchen, weitere zelluläre Bestandteile und/oder andere schweren Bestandteile, z.B. Fibrin. Das Abtrennen der zellulären und anderen schweren Bestandteile kann mittels Zentrifugation oder anderen in der biotechnologischen und medizinischen Technik genutzten Verfahren, die dem Fachmann bekannt sind, erfolgen.

Somit betrifft die vorliegende Erfindung nicht nur die Verwendung von Vollblut, sondern auch die Verwendung von Blutplasma für die Aufzucht von Fliegenlarven. In einer bevorzugten Ausführungsform wird Vollblut verwendet, besonders bevorzugt frisch gesammeltes Vollblut.

Sofern gelagertes Blut verwendet wird, kann es vor seiner Verwendung kalt, das heißt bei Temperaturen von 12°C bis 0 °C, bevorzugt bei Temperaturen von 4 °C +/- 2 °C, oder tiefkalt, das heißt bei Temperaturen von 0 °C bis -100 °C, bevorzugt bei Temperaturen von -5 °C bis -25 °C gelagert worden sein. Das Blut kann vor seiner Lagerung auch erhitzt worden sein, indem es Temperaturen von 40 °C - 90 °C, bevorzugt Temperaturen von 50 °C - 85 °C, länger, kurzzeitig oder ultrakurz (pasteurisiert) ausgesetzt wird.

Ganz besonders bevorzugt wird frisches, nicht gelagertes oder erhitztes Blut verwendet.

Blut gerinnt, sofern die Gerinnung nicht unterbunden wird. Im Rahmen der Erfindung kann sowohl geronnenes als auch ungeronnenes Blut verwendet werden. Hierfür können dem Blut gezielt Substanzen zugesetzt werden, die die Blutgerinnung verlangsamen oder beschleunigen.

In einer Ausführungsform wird die Blutgerinnung bei der Sammlung des Blutes verlangsamt oder gestoppt. Dies kann durch Zugabe verschiedener Substanzen erreicht werden. Vorzugsweise wird dem Blut ein Komplexbildner zugesetzt, der die Calcium-Katalyse der Blutgerinnung durch Komplexierung des Calciums stoppt. Beispiele für Substanzen, mit denen die Blutgerinnung verlangsamt oder gestoppt werden kann, sind Zitronensäure und Ethylendiamintetraessigsäure (EDTA).

Substanzen, die Calciumionen abgeben können, um die Gerinnung zu beschleunigen, können die Calciumsalze der bekannten Mineralsäuren oder die Calciumsalze organischer Säuren, beispielsweise Calciumoxid oder Calciumhydroxid, umfassen. Vorzugsweise werden Calciumsalze von Alginaten oder anderen Ionentauschern auf natürlicher oder synthetischer Basis verwendet.

Da geronnenes Blut verwendet werden kann, umfaßt die vorliegende Erfindung auch die Verwendung von menschlichem Blutserum für die Aufzucht von Fliegenlarven. Als Blutserum wird Blutplasma bezeichnet, aus dem das für die Gerinnung bedeutsame Fibrinogen entfernt wurde. Wie die Abtrennung der zellulären Bestandteile kann auch die Abtrennung des Fibrinogens aus dem Blut oder Blutplasma, nach seiner Umwandlung zu Fibrin und dessen Aggregation, mittels Zentrifugation oder anderen in der biotechnologischen und medizinischen Technik genutzten Verfahren, die dem Fachmann bekannt sind, erfolgen.

Gegenstand der vorliegenden Erfindung ist somit die Verwendung von humanem Blut, humanem Blutplasma oder humanem Blutserum als Nährsubstrat für die Aufzucht von Fliegenlarven, insbesondere von Larven der Fleischfliegen, wobei das Nährsubstrat keine komplexen Medienbestandteile enthält.

Das erfindungsgemäße Nährsubstrat enthält zwar keine komplexen Medienbestandteile, dem Blut, Blutplasma oder Blutserum können jedoch chemisch definierte Substanzen vorab oder später zugesetzt werden.

Chemisch definierte Substanzen, die dem Blut, Blutplasma oder Blutserum zugesetzt werden können, sind zum Beispiel Enzyme, Co-Enzyme, Aminosäuren, Zucker, Nukleobasen, Nukleoside, Nukleotide, Vitamine, Spurenelemente, Mineralstoffe und Antibiotika. Diese Substanzen werden dem Blut, Blutplasma oder Blutserum in einer Menge zugesetzt, die zu einer für die Larven ungiftigen Konzentrationen im Nahrungssubstrat führt.

Beispiele für geeignete Enzyme, die dem Blut, Blutplasma oder Blutserum zugesetzt werden können, sind Alkohol-Dehydrogenase, Aldehyd-Dehydrogenase, Aminopeptidase M, α-Amylase, β-Amylase, Amyloglucosidase, Bromelain, Carboxypeptidase A, Carboxypeptidase B, Carboxypeptidase Y, Katalase, Cholinesterase, α-Chymotrypsin, Chymotrypsinogen A, Kollagenase, Kreatinkinase, Desoxyribonuclease I, Diaphorase, Elastase, Formiat-Dehydrogenase, Glucose-Dehydrogenase, Glucose-Oxidase,

Glucose-6-phosphat-Dehydrogenase, α-Glucosidase, β-Glucuronidase/ Arylsulfatase, Glutamat-Dehydrogenase, Glycerol-3-phosphat-Dehydrogenase, Hexokinase, Hyaluronidase, Lactat-Dehydrogenase, Leucine-Aminopeptidase, Lipase, Lysozym, Malat-Dehydrogenase, Myokinase, Neuraminidase, Papain, Pepsin, Pepsinogen, Peroxidase, alkalische Phosphatase, saure Phosphotase, Phosphodiesterase I, Phosphodiesterase II, Phosphoglucose-Isomerase, Pronase E, Proteinase K, Pyruvat-Kinase, Ribonuclease, Saccharase, Sorbitol-Dehydrogenase, Thermolysin, Thrombin, Trypsin, Trypsin-Inhibitor, Trypsinogen, Urease und Uricase.

Beispiele für geeignete Co-Enzyme, die dem Blut, Blutplasma oder Blutserum zugesetzt werden können, sind Adenosine-3',5'-monophosphat, Adenosine-5'-diphsophat, Adenosine-5'-monophosphat, Adenosine-5'-triphosphat, Thiaminpyrophosphat, Dihydronicotinamid-adenin-dinucleotid, Dihydronicotinamid-adenin-dinucleotidphosphat, Guanosin-5'-triphosphat, Nicotinamid-adenin-dinucleotid, Nicotinamid-adenin-dinucleotidphosphat, Pyridoxal-5'-phosphat, Pyridoxamin-5'-phosphat und Uridin-5'-diphosphat-Glucose.

Beispiele für geeignete Aminosäuren, die dem Blut, Blutplasma oder Blutserum zugesetzt werden können, sind N-Acetyl-L-cystein, N-Acetyl-D-tryptophan, D-Alanin, L-

Alanin, β-Alanine, 4-Aminohippursäure, 5-Aminolevulinsäure, L-Arginin, D-Asparagin, L-Asparagin, Asparaginsäure, L-Citrullin, L-Cystein, L-Cystin, 3,4-Dihydroxy-L-phenylalanin, Ethylglycinat, D-Glutaminsäure, L-Glutaminsäure , L-Glutamin, Glycin, Glycylglycin, Hippursäure, Histidin, L-Hydroxyprolin, 3-Iodo-L-tyrosin, L-Isoleucin, D-Leucin, L-Leucin, L-Lysin, L-Methionin, L-Ornithin, D-Phenylalanin, L-Phenylalanin, L-Prolin, D-Serin, L-Serin, Serotonin-Creatininsulfat, DL-Threonin, L-Threonin, L-Thyroxin, D-Tryptophan, L-Tryptophan, L-Tyrosin, D-Valin und L-Valin.

Beispiele für geeignete Zucker, die dem Blut, Blutplasma oder Blutserum zugesetzt werden können, sind D-Arabin-N-Acetylneuraminsäure, Adonitol, Amylose, Arabinose, Cellobiose, 2-Deoxy-D-glucose, 2-Deoxy-D-ribose, Dulcitol, meso-Erythritol, D-Fructose, D-Fucose, D-Galactosamin-hydrochlorid, D-Galactose, D-Galacturonsäure, Gluconsäure-6-phosphat, D-Gluconat, Gluconolacton, D-Glucosamin-hydrochloride, D-Glucose, Glucose-1-phosphat, Glucose-6-phosphat, D-Glucurono-6-lacton, DL-Glycerinaldehyd, Glycerol, Glycogen, myo-Inotisol, myo-Inosit, Inulin, Lactose, Lactulose, Maltose, D-Mannitol, D-Mannose, Mellibiose, Raffinose, Rhamnose, D-Ribose, D-Sorbitol, L-Sorbose, Stärke, Saccharose, Sucrose, Trehalose, Xylitol und D-Xylose.

Beispiele für geeignete Nukleobasen, Nukleoside und Nukleotide, die dem Blut, Blutplasma oder Blutserum zugesetzt werden können, sind Adenin, Adenosin, Adenosin-3',5'-monophosphat, Adenosin-5'-diphosphat, Adenosin-5'-monophosphat, Adenosin-5'-triphosphat, Cytidin-2',3'-monophosphat, Cytosin, 2-Deoxythymidin, Guanin, Guanosin-5'-triphosphat, Hypoxanthin, Inosin, Thymin, Uracil, Uridin-5'-diphosphoat-Glucose und Xanthine.

Beispiele für geeignete Vitamine, die dem Blut, Blutplasma oder Blutserum zugesetzt werden können, sind L(+)Ascorbinsäure, D(+)Biotin, β-Carotin, Folsäure, Menachinon, Nicotinamid, Nicotinsäure, D(+)Pantothensäure, Pyridoxalhydrochlorid, Pyridoxamin-dihydrochlorid, Pyridoxolhydrochlorid, Riboflavin, Thiamin, D∼-α-Tocopherol, DL-α-Tocopherol-acetat, Tocopherol-Isomere (alpha, beta, gamma, delta), Vitamin A-acetat Vitamine A-palmitat, Vitamin B₁₂, Vitamin D₂, Vitamin D₃, Vitamin K₁, Carnitin und Cholesterol.

Beispiele für geeignete Spurenelemente und Mineralstoffe, die dem Blut, Blutplasma oder Blutserum zugesetzt werden können, sind Ammonium, Aluminium, Arsen, Bor, Brom, Chlor, Eisen, Iod, Kobalt, Kupfer, Lithium, Magnesium, Mangan, Molybdän, Nickel, Nitrat, Phosphat, Selen, Silizium, Sulfat, Vanadium, Wolfram und Zink.

Beispiele für Antibiotika , die dem Blut, Blutplasma oder Blutserum zugesetzt werden können, sind Penicilline, beispielsweise Penicillin G, Penicillin V, Oxacillin, Amoxicillin, Mezlocillin, Ticarcillin, Azlocillin, Piperacillin oder Apalcillin, Cephalosporine wie Cephalexin, Cefaclor, Cetadroxil, Cefalothin, Cefazolin, Ceturoxim, Cefoxlin, Cefamandol, Cefotiam, Cefotaxim, Cefmenoxim, Ceftizoxim, Ceftriaxon, Latamoxef, Ceftazidim, Cefsulodin, Cephalosporin oder Cettazidim, β-Lactam-Antibiotika, zum Beispiel Carbapeneme, Imipenem, Monobactamem oder Azthreonam, Aminoglykoside wie Streptomycin, Neomycin, Gentamicin, Tobramycin, Netilmicin oder Amikacin, Tetracycline, beispielsweise Tetracyclin, Doxycyclin oder Minocyclin, Chloramphenicol, Makrolide wie Erythromycin, Lincomycine, zu denen Lincomycin und Clindamycin gehören, Polymyxine wie Polymyxin B oder Colistin, Sulfonamide wie Sulfanilamid, Nitrofurane, zum Beispiel Nitrofurantoin, Nitro-Imidazole, nämlich Metronidazol, Chinolone, die Nalidixinsäure-Präparate, Norfloxacin-Präparate, Ofloxacin, Ciprofloxacin und Enoxacin umfassen, Rifamycine wie Rifampicin, Vancomycin, Fusidinsäure und Fosfomycin.

In einer besonders bevorzugten Ausführungsform wird das Blut des zu behandelnden Patienten für die Aufzucht der Calliphoriden-Larven verwendet, die für seine Behandlung eingesetzt werden sollen. Durch Verwendung des Blutes des zu behandelnden Patienten selbst können keine neuen Krankheitserreger auf den Patienten übertragen werden, mit denen der Patient nicht bereits infiziert ist. Bei einer gezielten Zucht der Larven für die Behandlung eines spezifischen Patienten wird lediglich eine kleine Menge (wenige Milliliter) seines Blutes benötigt, um eine therapeutisch sinnvolle Anzahl an Larven herstellen zu können.

Für eine möglichst einfache Herstellung der Larven, das heißt für ihre problemlose Aufzucht und Aufbereitung für die medizinische Anwendung, hat sich das folgende Verfahren als besonders vorteilhaft herausgestellt:

Desinfizierte Eier von Fliegen werden in einer hartwandigen oder aus flexiblem Material gefertigten Vorrichtung zur Aufzucht von Fliegenlarven vorgelegt. Es ist jedoch auch möglich, das Nährsubstrat vorzulegen und die Eier der Fliegen als letztes in die Vorrichtung einzubringen. Das Material der Vorrichtung ist für Maden und Mikroorganismen undurchdringbar. Darüber hinaus ist das Material vorzugsweise beständig gegen Sterilisation durch Dampf, Hitze oder Strahlung und Feuchtigkeit. Das Material weist bevorzugt eine glatte Oberflächenstruktur auf und gibt keine Schadstoffe ab, so daß Eier und Maden im Aufzuchtbehälter nicht geschädigt werden. Es können beispielsweise Vorrichtungen aus Glas, Email oder Kunststoff verwendet werden.

Diese Vorrichtung ist ein Zuchtbehälter, der mit einer oder mehreren verschließbaren Öffnungen versehen ist. Jede der Öffnungen ist mit einem Deckel verschlossen, wobei der oder mindestens einer der Deckel einen Gasaustausch zuläßt, Bakterien oder andere pathogene Organismen aber nicht passieren läßt, und die Möglichkeit bietet, Flüssigkeiten unter sterilen Bedingungen zudosieren zu können, beispielsweise indem er ein Septum aufweist. Bei dem Deckel kann es sich um einen Schraubverschluß, einen Drehverschluß, einen Klemmverschluß, einen Spannverschluß oder einem anderen aus dem Stand der Technik bekannten Verschluß handeln

Durch diese Art des Verschlusses kann bei Bedarf Blut zugegeben werden, und die schlüpfenden Larven können sich unter Brutbedingungen innerhalb von 1 bis 5 Tagen zu Larven entwickeln, die therapeutisch genutzt werden können.

In einer besonders vorteilhaften Ausführungsform weist die Vorrichtung in ihrem Inneren eine saugfähige Unterlage auf, mit der verhindert wird, daß die Eier oder die Larven in das Blut fallen und ertrinken. Für die saugfähige Unterlage können natürliche saugfähige Materialien verwendet werden, beispielsweise Watte, Alginate, Cellulosen, Pektine oder Chitosane, oder synthetische saugfähige Materialien wie aus Polymeren gewonnene Schwämme, Hohlkörper, Fasern oder Ionentauscher. Die Art des Polymers ist von untergeordneter Bedeutung, prinzipiell können alle physiologisch unbedenklichen Polymere verwendet werden. Vorzugsweise werden Polyurethane, Polyacrylate, Polyester, Polyvinylchloride, Polyalkohole oder andere mit polaren Gruppen ausgestattete Polymere verwendet.

Das saugfähige Material im Zuchtbehälter kann unbehandelt verwendet werden oder mit verschiedenen Substanzen vorbehandelt werden, bevor es zum Einsatz kommt. So können bestimmte Enzyme, Co-Enzyme, Aminosäuren, Zucker, Nucleobasen, Nucleoside, Nucleotide, Vitamine, Spurenelemente, oder andere der optimalen Ernährung der Larven dienende Stoffe zugesetzt werden. So kann das saugfähige Material mit den Substanzen vorbehandelt werden, die auch dem Blut, Blutplasma oder Blutserum zugesetzt werden können. Vorzugsweise wird das saugfähige Material mit einer Substanz vorbehandelt, die die Eigenschaften des Blutes verändern, zum Beispiel indem es die Blutgerinnung beschleunigt oder verlangsamt. Vorteilhaft ist ein Zusatz von Calciumionen abgebenden Salzen, wenn zuvor das Blut unter Verwendung von Komplexbildnern des Calciums zur Unterdrückung der Blutgerinnung gesammelt wurde. So kann in der Kombination von Komplexbildnern beim Sammeln des Blutes und Calciumionen auf dem saugfähigen Material die Blutgerinnung nahezu beliebig eingestellt werden, so daß die Larven schnell das Nahrungssubstrat aufnehmen können, ohne im diesem zu ersticken oder zu ertrinken.

Besonders bevorzugt werden Substanzmischungen und Ionenbesatz in der Art verwendet, daß das saugfähige Material fest an der Innenwand des Zuchtbehälters hält und anfänglich leicht von den Larven durchdrungen werden kann. So können bevorzugt zum Einsatz kommende Ionentauscher Calcium abgeben wie aufnehmen, mit anderen Ionen, beispielsweise Kalium-, Natrium- oder Zinkionen, versetzt sein und durch den Stoffwechsel der Larven entstehendes Ammonium aufnehmen. Der/die Ionenaustauscher können allein oder im Gemisch mit anderen Polymeren vorliegen, um die physikalische Struktur des saugfähigen Materials zu optimieren. Besonders bevorzugte Ionenaustauscher sind Calciumalginate, die bei Entzug des Calciums vergelen. Dies kann ein gewünschter oder ungewünschter Prozeß sein, der jedoch durch die Menge der verwendeten Komplexbildner sowie durch Zusatz von anderen Polymeren ohne ionentauschende Funktion leicht gesteuert werden kann.

Damit das saugfähige Material durch die Aktivität der Larven nicht in Einzelteile zerfällt, wird es bei einer bevorzugten Ausführungsform durch ein für die Larven durchdringbares oder undurchdringbares Netz oder Gaze zusammengehalten und/oder durch eine Trennschicht von den Fliegen-Larven getrennt. Die Trennschicht ist in jedem Fall geeignet, die von den Larven ausgeschiedene Säfte sowie Blut oder andere Flüssigkeiten passieren zu lasen, damit sich die Larven leicht ernähren können. Allerdings können die Larven nicht durch die Trennschicht in das saugfähige Material einwandern. Als Materialien für die Trennschicht kommen beispielsweise Gaze, perforierte Folien oder Membranen in Betracht.

Die Eier der Fliegen können prinzipiell an jedem beliebigen Ort innerhalb des Zuchtbehälters plaziert werden, solange die Eier dort nicht durch Austrocknung oder mechanische Einwirkung zerstört werden. Vorzugsweise werden die Eier in der Nähe des Blutes, Blutplasmas oder Blutserums plaziert, damit die Larven unmittelbar nach ihrem Schlüpfen Nahrung finden.

Die Eier können lose in den Zuchtbehälter eingebracht werden oder in einem Beutel. Da die Eier bei loser Lagerung im Zuchtbehälter durch mechanische Stöße während des Transportes ungewollt verteilen werden können, wird ein Einbringen der Eier in einem Beutel bevorzugt. Dieser Beutel besteht aus einer Gaze, welche die Eier zurückhält, die schlüpfenden Larven aber passieren läßt. Die Verwendung eines solchen Beutels hat weiterhin den Vorteil, daß die Eihüllen und blinde Eier später leichter von den Larven separiert werden können, weil sie im Beutel verbleiben. Der Beutel selbst kann frei im Zuchtbehälter liegen oder an einem gewünschten Ort an der Innenwand fixiert werden.

Wenn die Larven die gewünschte Größe erreicht haben, müssen sie vom restlichen Blut, Eihüllen, blinden Eiern und ggf. saugfähigem Material abgetrennt werden. Dazu wird bevorzugt der Zuchtbehälter mit einer Separationsflüssigkeit gefüllt, die so gewählt ist, daß sich die Larven und die restlichen Bestandteile aus der Larvenkultur aufgrund von Dichteunterschieden auf entgegengesetzten Seiten der Separationsflüssigkeit anreichern. Vorzugsweise wird als Separationsflüssigkeit eine Salzlösung verwendet, die in Konzentration und Zusammensetzung so gewählt ist, daß der gewünschte Effekt erreicht wird. Eine gute Trennung von Larven und Nährsubstrat erzielt man mit physiologischer Kochsalzlösung (0,9% NaCl). Der Separationsflüssigkeit können auch weitere Substanzen zugesetzt werden, zum Beispiel antimikrobiell wirksame Substanzen, desinfizierende Substanzen oder sonstige Zusätze, die die Trennung oder Anwendung der Larven erleichtern. Bevorzugt kommt Polyhexanid zum Einsatz, das in einer Konzentration von 0,001 bis 0,1 %, besonders bevorzugt 0,01 bis 0,5 % verwendet werden kann.

Mit menschlichem Blut, Blutplasma oder Blutserum, vorzugsweise mit dem oben beschriebenen Verfahren und der beschriebenen Vorrichtung, können nicht nur die Larven der Sarcophagidae (Fleischfliegen), insbesondere aus der Gattung *Sarcophaga,* und der Calliphoridae (Schmeißfliegen), insbesondere aus den Gattungen *Lucilia, Phormia* und *Calliphora*, sondern auch die Larven von Fliegen der Gattung Musca aufgezogen werden. Besonders bevorzugt kommen die Eier von *Lucilia sericata* zum Einsatz.

Soweit nicht anders angegeben, wurde in den nachfolgenden Versuchen Blutagar der Firma Merck (Darmstadt) (Nr. 1.13414.0001 / Blut: Hammel / typische Zusammensetzung (g/L): Nährsubstrat (Hefeextrakt, Peptone, Leberhydrolysat) 23,0; Natriumchlorid 5,0; Agar-Agar 12,0) verwendet. Je Versuchsansatz wurden 90 - 150 Eier eingesetzt.
Die verwendeten Materialien wurden kühl gelagert oder tiefgefroren und das Blut mit Citrat gehemmt.
Bei der Wundwatte handelte es sich um Standard-Watte der Firma Hartmann.

### Beispiel 1 (Vergleichsbeispiel aus dem Stand der Technik)

In Reagenzgläsern mit gasdurchlässigem bakteriendichten Deckel wurden Stücke von Blut-Agar eingebracht, mit desinfizierten Eiern von *Lucilia sericata* besetzt und 48 h Brutbedingungen ausgesetzt.

Als Maß für die Wundreinigung wurde anschließend Schweineleber in den Zuchtbehälter eingebracht und die Abnahme des Lebergewebes in den folgenden zwei Tagen (48 bis 96 h) beobachtet.

Die Entwicklung der Larven und der Abbau des Lebergewebes sind Tabelle 1 zu entnehmen.

**Tabelle 1**

| Medium: Blutagar | lebende Larven nach 48 h Brutzeit | Gewichtsabnahme des Lebergewebes |
|---|---|---|
| Röhrchen 1 | 45,7% | 25,6% |
| Röhrchen 2 | 40,0% | 32,1% |
| Röhrchen 4 | 62,5% | 45,1% |
| Röhrchen 5 | 44,0% | 51,1% |
| Röhrchen 6 | 63,3% | 29,9% |
| Röhrchen 7 | 46,0% | 39,4% |
| Röhrchen 8 | 61,7% | 29,6% |
| Röhrchen 9 | 1,3% | - |
| Röhrchen 10 | 38,0% | 27,2% |
| | 45,3% | 35,0% |

### Beispiel 2

In Reagenzgläsern mit gasdurchlässigem bakteriendichten Deckel und Wundwatte am Boden wurde menschliches Blut eingebracht, mit desinfizierten Eiern von *Lucilia sericata* besetzt und 48 h Brutbedingungen ausgesetzt.

Als Maß für die Wundreinigung wurde anschließend Schweineleber in den Zuchtbehälter eingebracht und die Abnahme des Lebergewebes in den folgenden zwei Tagen (48 bis 96 h) beobachtet.

Die Entwicklung der Larven und der Abbau des Lebergewebes sind Tabelle 2 zu entnehmen.

**Tabelle 2**

| Medium: Humanblut | lebende Larven nach 48 h Brutzeit | Gewichtsabnahme des Lebergewebes |
|---|---|---|
| Röhrchen 1 | 70,0% | 65,2% |
| Röhrchen 2 | 41,0% | 50,8% |
| Röhrchen 4 | 35,6% | 52,9% |
| Röhrchen 5 | 27,0% | 49,3% |
| Röhrchen 6 | 44,0% | 66,7% |
| Röhrchen 7 | 63,0% | 51,5% |
| Röhrchen 8 | 46,0% | 47,0% |
| Röhrchen 9 | 50,0% | 35,2% |
| Röhrchen 10 | 88,0% | 43,4% |
| | 51,6% | 51,3% |

Es ist erkennbar, daß die Larven auf menschlichem Blut mit höherer Rate überleben und bessere wundreinigende Eigenschaften aufweisen, als die Larven, die auf Blut-Agar gemäß Beispiel 1 gezogen wurden.

### Beispiel 3

In Reagenzgläsern mit gasdurchlässigem bakteriendichten Deckel und Wundwatte am Boden wurde menschliches Blut eingebracht, mit desinfizierten Eiern von *Lucilia sericata* besetzt und Brutbedingungen ausgesetzt. Dem Blut wurde zuvor eine der in Tabelle 3 aufgeführten Komponenten zugemischt.

**Tabelle 3**

| Zusatz von | Konzentration | Beobachtung |
|---|---|---|
| Zitronensäure | 0,01% | Madenwachstum gleich oder besser Vergleichsversuch |
| L(+)Ascorbinsäure | 0,01% | Madenwachstum gleich oder besser Vergleichsversuch |
| Lipase | 0,001% | Madenwachstum gleich oder besser Vergleichsversuch |
| Glycerin | 0,01% | Madenwachstum gleich oder besser Vergleichsversuch |
| Saccharose | 0,01% | Madenwachstum gleich oder besser Vergleichsversuch |
| Glukose | 0,01% | Madenwachstum gleich oder besser Vergleichsversuch |
| Nicotinamid adenine dinucleotid | 0,001% | Madenwachstum gleich oder besser Vergleichsversuch |
| Tocopherol isomere | 0,001% | Madenwachstum gleich oder besser Vergleichsversuch |
| Zinksulfat | 0,001% | Madenwachstum gleich oder besser Vergleichsversuch |
| Guanin | 0,01% | Madenwachstum gleich oder besser Vergleichsversuch |
| Glycin | 0,01% | Madenwachstum gleich oder besser Vergleichsversuch |
| myo-Inosit | 0,01% | Madenwachstum gleich oder besser Vergleichsversuch |
| ohne Zusatz | 0 | Vergleichsversuch ohne Zusatz |

Die Ergebnisse dieser semiquantitativen Versuchsreihe zeigen, daß dem Blut verschiedene chemisch definierte Substanzen zugesetzt werden können, ohne daß die Entwicklung der Calliphoriden-Larven beeinträchtigt wird. Vielmehr führt der Zusatz dieser Substanzen zu einer Verbesserung des Madenwachstums.

## Patentansprüche

1. Verwendung von menschlichem Blut, menschlichem Blutplasma oder menschlichem Blutserum zur Aufzucht von Fliegen-Larven.

2. Verwendung nach Anspruch 1, **dadurch gekennzeichnet, daß** dem Blut, Blutplasma oder Blutserum keine komplexen Medienbestandteile zugesetzt sind.

3. Verwendung nach Anspruch 1 oder 2, **dadurch gekennzeichnet, daß** dem Blut, Blutplasma oder Blutserum mindestens eine Substanz aus der Gruppe von Substanzen zugesetzt wird, die aus Enzymen, Co-Enzymen, Aminosäuren, Zuckern, Nucleobasen, Nucleosiden, Nucleotiden, Vitaminen, Spurenelementen, Mineralstoffen und Antibiotika besteht.

4. Verwendung nach einem der vorhergehenden Ansprüche, **dadurch gekennzeichnet, daß** das Blut, Blutplasma oder Blutserum erhitzt wurde.

5. Verwendung nach einem der vorhergehenden Ansprüche, **dadurch gekennzeichnet, daß** das Blut, Blutplasma oder Blutserum kalt oder tiefkalt gelagert wurde.

6. Verwendung nach einem der vorhergehenden Ansprüche, **dadurch gekennzeichnet, daß** die Gerinnung des Blutes oder Blutplasmas gefördert, gehemmt oder unterbunden wurde.

7. Verwendung nach einem der vorhergehenden Ansprüche, **dadurch gekennzeichnet, daß** es sich bei den Fliegen-Larven um Larven aus einer der Familien der Muscidae, Calliphoridae oder Sarcophagidae handelt.

8. Verwendung nach Anspruch 7, **dadurch gekennzeichnet, daß** es sich um Fliegen aus einer der Gattungen *Musca*, *Sarcophaga, Phormia*, *Calliphora* oder *Lucilia* handelt, vorzugsweise um *Lucilia sericata.*

9. Vorrichtung zur Aufzucht von Fliegen-Larven, umfassend einen Behälter aus einem hartwandigen oder flexiblen Material, das für Maden und Mikroorganismen undurchdringbar ist, der mindestens eine Öffnung aufweist, durch die Flüssigkeit in den Behälter eingebracht werden kann, wobei jede Öffnung des Behälters mit einem verschließbaren Deckel versehen ist, und wobei der Deckel oder mindestens einer der Deckel einen Gasaustausch zwischen dem Innenraum des Behälters und der Umgebung ermöglicht, der ein Einbringen der Flüssigkeit(en) ermöglicht, ohne daß der Deckel geöffnet werden muß, der aber für Mikroorganismen undurchdringbar ist.

10. Vorrichtung nach Anspruch 9, **dadurch gekennzeichnet, daß** der Behälter ein saugfähiges Material enthält.

11. Vorrichtung nach Anspruch 10, **dadurch gekennzeichnet, daß** das saugfähige Material ein natürliches Material, das vorzugsweise aus der Gruppe von Materialien ausgewählt ist, die aus Watte, Alginaten, Cellulosen, Pektinen und Chitosanen besteht, oder ein synthetisches Material ist, das vorzugsweise aus der Gruppe von Materialien ausgewählt ist, die aus Polymeren hergestellte Schwämme, Hohlkörper, Fasern und Ionenaustauscher umfaßt.

12. Vorrichtung nach Anspruch 10 oder 11, **dadurch gekennzeichnet daß** sich das saugfähige Material in einem Netz oder einem Beutel aus Gaze, perforierter Folie oder semipermeablen Membranen befindet, das/der Blut passieren läßt, aber Maden zurückhält.

13. Vorrichtung nach einem der Ansprüche 9 bis 12, **dadurch gekennzeichnet, daß** das Blut, Blutplasma, Blutserum und saugfähige Material durch eine Trennschicht vom restlichen Lumen des Behälters getrennt ist.

14. Vorrichtung nach Anspruch 13, **dadurch gekennzeichnet, daß** die Trennschicht ein Netz, eine Gaze, eine perforierte Folien oder eine Membran ist, und Blut passieren läßt, aber Maden zurückhält.

15. Verfahren zur Aufzucht von Fliegen-Larven, **dadurch gekennzeichnet, daß** Fliegen-Eier in ein Behältnis eingebracht werden, in das menschliches Blut, menschliches Blutplasma oder menschliches Blutserum zugegeben wird, oder in dem sich bereits menschliches Blut, menschliches Blutplasma oder menschliches Blutserum befindet, das Behältnis unter Brutbedingungen inkubiert wird, bis die Larven schlüpfen und die für ihre medizinische Anwendung erforderliche Größe haben, und anschließend die Larven vom restlichen Inhalt des Behältnisses separiert werden.

16. Verfahren nach Anspruch 15, **dadurch gekennzeichnet, daß** die Eier in einem Beutel in das Behältnis eingebracht werden, wobei der Beutel aus einer Gaze besteht, die die Eier nicht durchläßt, die die schlüpfenden Larven jedoch nicht zurückhält.

17. Verfahren nach Anspruch 16, **dadurch gekennzeichnet, daß** der Beutel an der Innenwand des Behältnisses befestigt wird.

18. Verfahren nach einem der Ansprüche 15 bis 17, **dadurch gekennzeichnet, daß** die Separation mit Hilfe einer Separationsflüssigkeit erfolgt, bei der sich die Larven und die restlichen Bestandteile auf den gegenüberliegenden Seiten der Separationsflüssigkeit anreichern.

19. Verfahren nach Anspruch 18, **dadurch gekennzeichnet, daß** die Separationsflüssigkeit eine Salzlösung ist, vorzugsweise physiologische Kochsalzlösung.

20. Verfahren nach Anspruch 18 oder 19, **dadurch gekennzeichnet, daß** die Separationsflüssigkeit eine antimikrobiell wirksame oder desinfizierende Substanz enthält, vorzugsweise Polyhexanid.

21. Verfahren nach einem der Ansprüche 15 bis 20, **dadurch gekennzeichnet, daß** eine Vorrichtung nach einem der Ansprüche 9 bis 14 verwendet wird.

22. Verfahren nach einem der Ansprüche 15 bis 21, **dadurch gekennzeichnet, daß** es sich bei den Fliegen-Larven um Larven aus einer der Familien der Muscidae, Calliphoridae oder Sarcophagidae handelt.

23. Verfahren nach Anspruch 22, **dadurch gekennzeichnet, daß** es sich bei den Fliegen um Fliegen aus einer der Gattungen *Musca, Sarcophaga, Phormia, Calliphora* oder *Lucilla* handelt, vorzugsweise um *Lucilia sericata.*
